# EUROPÄISCHE PATENTANMELDUNG

(11) **EP 4 559 508 A1**
(43) Veröffentlichungstag der Anmeldung: **28.05.2025**
(21) Anmeldenummer: 24213553.1
(22) Anmeldetag: 18.11.2024
(51) Int. Cl.: A61N 1/05, A61N 1/36

(54) **SYSTEM ZUR ELEKTRISCHEN NEUROSTIMULATION**

(30) Priorität: 24.11.2023 DE 102023132855
(71) Anmelder: B. Braun Melsungen AG, 34212 Melsungen (DE)
(72) Erfinder: Wildhagen, Jens, 30659 Hannover (DE)
(74) Vertreter: Patentanwälte Ruff, Wilhelm, Beier, Dauster & Partner mbB

(57) **Zusammenfassung**

Ein System aufweisend einen Stimulator mit einem längserstreckten Stimulatorschaft, der ein distales Stimulatorende mit mehreren Elektroden aufweist, die entlang einer Längsachse des Stimulatorschafts benachbart angeordnet und zur Abgabe elektrischer Stimuli an ein den Stimulatorschaft umgebendes Körpergewebe eingerichtet sind, eine Steuereinrichtung, die mit den mehreren Elektroden verbunden und zum Steuern der Abgabe der elektrischen Stimuli eingerichtet ist, wobei die mehreren Elektroden mittels der Steuereinrichtung voneinander unabhängig aktivierbar und deaktivierbar und damit zur Ausbildung entlang der Längsachse unterschiedlicher Elektrodenaktivierungsmuster ansteuerbar sind, ist bekannt.

Erfindungsgemäß ist eine Ermittlungseinrichtung vorhanden und zum Ermitteln einer axialen Dislokation des Stimulatorschafts eingerichtet, wobei die Steuereinrichtung mit der Ermittlungseinrichtung verbunden und zum axialen Verschieben des Elektrodenaktivierungsmusters mittels einer Veränderung der Aktivierung und Deaktivierung der Elektroden in Abhängigkeit der ermittelten axialen Dislokation eingerichtet ist, um die Abgabe der elektrischen Stimuli an die axiale Dislokation des Stimulatorschafts örtlich anzupassen.

## Beschreibung

Die Erfindung betrifft ein System zur elektrischen Neurostimulation aufweisend einen Stimulator und eine Steuereinrichtung. Der Stimulator weist einen längserstreckten Stimulatorschaft auf, der ein distales Stimulatorende mit mehreren Elektroden aufweist. Die mehreren Elektroden sind entlang einer Längsachse des Stimulatorschafts benachbart angeordnet und zur Abgabe elektrischer Stimuli an ein den Stimulatorschaft umgebendes Körpergewebe eingerichtet. Die Steuereinrichtung ist mit den mehreren Elektroden verbunden und zum Steuern der Abgabe der elektrischen Stimuli eingerichtet. Die mehreren Elektroden sind mittels der Steuereinrichtung voneinander unabhängig aktivierbar und deaktivierbar und damit zur Ausbildung entlang der Längsachse unterschiedlicher Elektrodenaktivierungsmuster ansteuerbar.

Derartige Systeme sind im Bereich der Medizintechnik allgemein bekannt und zur Verwendung bei einer Schmerztherapie vorgesehen. Bei einer als peripherer Nervenblock (PNB) bezeichneten Schmerztherapie wird der Stimulator so weit distal vorgeschoben, dass das distale Stimulatorende nahe dem zu behandelnden Nerv positioniert ist. Nach der Positionierung des Stimulatorschafts werden diejenigen Elektroden zur Aktivierung ausgewählt, die unter Ausbildung eines bestimmten Elektrodenaktivierungsmusters ein optimales Stimulationsergebnis erzielen. Ein optimales Stimulationsergebnis kann beispielsweise durch eine maximale Schmerzreduktion charakterisiert sein. Die Auswahl des Elektrodenaktivierungsmusters kann zum Beispiel anhand einer Patientenrückmeldung erfolgen. Ein derartiges System ist beispielsweise aus der US 8,005,538 B2 bekannt.

Aufgabe der Erfindung ist es, ein System der eingangs genannten Art bereitzustellen, das eine verbesserte Schmerztherapie ermöglicht.

Diese Aufgabe wird dadurch gelöst, dass eine Ermittlungseinrichtung vorhanden und zum Ermitteln einer axialen Dislokation des Stimulatorschafts eingerichtet ist, wobei die Steuereinrichtung mit der Ermittlungseinrichtung verbunden und zum axialen Verschieben des Elektrodenaktivierungsmusters mittels einer Veränderung der Aktivierung und Deaktivierung der Elektroden in Abhängigkeit der ermittelten axialen Dislokation eingerichtet ist, um die Abgabe der elektrischen Stimuli an die axiale Dislokation des Stimulatorschafts örtlich anzupassen. Die Erfindung geht von der Überlegung aus, dass unbeabsichtigte axiale Bewegungen des Stimulatorschafts die Wirksamkeit der Schmerztherapie beeinträchtigen können. Ein unbeabsichtigtes proximales Herausziehen oder distales Hineinschieben des Stimulatorschafts führt zu einer Veränderung der Position der Elektroden in Bezug auf den zu stimulierenden Nerv. Ein zunächst ausgewähltes Elektrodenaktivierungsmusters kann bei Auftreten einer axialen Dislokation zu einer nur noch suboptimalen Stimulation des Nervs führen und dadurch die Wirksamkeit der Schmerztherapie beeinträchtigen. Um dem entgegenzuwirken, verschiebt die Steuereinrichtung bei Auftreten einer axialen Dislokation das Elektrodenaktivierungsmuster entlang der Längsachse des Stimulatorschafts. Das Elektrodenaktivierungsmuster wird der ermittelten axialen Dislokation gleichsam nachgeführt. Hierdurch wird erreicht, dass das Elektrodenaktivierungsmuster die ursprüngliche Position in Bezug auf das umgebende Körpergewebe und insbesondere den zu stimulierenden Nerv auch nach einer etwaigen Dislokation des Stimulatorschafts wieder einnimmt oder die ursprüngliche Position trotz der Dislokation beibehält oder eine Abweichung der nach der Dislokation eingenommenen Position von der ursprünglichen Position zumindest minimiert wird. Somit werden durch die erfindungsgemäße Lösung proximale Dislokationen des distalen Stimulatorendes ausgeglichen oder deren Beeinträchtigungen zumindest reduziert. Hierdurch ist eine stets optimale oder quasi-optimale Stimulation des umgebenden Körpergewebes bzw. des zu behandelnden Nervs gewährleistet, die gegenüber äußeren Einflüssen oder Bewegungen des Patienten robust ist. Das Verschieben des Elektrodenaktivierungsmusters zum Anpassen an die axiale Dislokation erfolgt insbesondere um eine Länge, die dem Abstand benachbarter Elektroden oder einem Vielfachen davon entspricht. Bei einer proximalen Dislokation des Stimulatorschafts wird das Elektrodenaktivierungsmuster distal verschoben. Bei einer distalen Dislokation des Stimulatorschafts wird das Elektrodenaktivierungsmuster proximal verschoben. Eine Abweichung der neu eingenommenen Position von der ursprünglichen Position ist unwesentlich, wenn trotz der Abweichung der gewünschte Effekt, insbesondere ein schmerzverringernder Effekt, des elektrischen Stimulus auf den Nerv bzw. das umgebende Körpergewebe erreicht wird. Mit Stimulus ist jede Art von über die Elektroden abgegebenem Strom und/oder an den Elektroden angelegter Spannung gemeint, insbesondere ein einzelner oder mehrere aufeinanderfolgende Strom- oder Spannungsimpulse oder eine anhaltende Abgabe von Strom oder ein anhaltendes Anlegen einer Spannung. Je enger benachbart die Elektroden entlang der Längsachse angeordnet sind und je größer die Anzahl der Elektroden ist, umso geringer kann eine Abweichung sein, welche die Position des Elektrodenaktivierungsmusters nach der Anpassung an die Dislokation relativ zur Position vor der Dislokation maximal aufweisen kann. Die Abweichung beträgt insbesondere maximal die Hälfte des Abstands zwischen zwei Elektroden. Vorzugsweise weist der Stimulator eine Vielzahl von Elektroden auf, vorzugsweise zwischen 5 und 15 Elektroden, jedoch mindestens drei Elektroden. Vorteilhaft nehmen die Elektroden einen von zwei Zuständen ein. In diesem Fall ist das Elektrodenaktivierungsmuster, gebildet aus der Abfolge aus aktivierten und deaktivierten Elektroden entlang der Längsachse des Stimulatorschafts, als Binärcode darstellbar. Beispielsweise können zur Abgabe eines elektrischen Stimulus zwei Elektroden im aktivierten Zustand zwischen sich eine Abfolge aus einem positiven Strom- oder Spannungsimpuls und einem negativen Strom- oder Spannungsimpuls, oder umgekehrt, abgeben (sog. Biphasen-Signal oder bipolares Impulssignal). Eine Elektrode im deaktivierten Zustand gibt dann entsprechend kein Signal ab. Alternativ hierzu kann zur Abgabe eines elektrischen Stimulus eine Elektrode im aktivierten Zustand ein Wechselspannungs- oder ein Wechselstromsignal abgeben, während die Elektrode im deaktivierten Zustand kein Signal abgibt. Die Ermittlungseinrichtung ist zum Ermitteln der axialen Dislokation des Stimulatorschafts eingerichtet. Hierfür kann grundsätzlich jedes zum Erfassen von Längen- oder Positionsänderungen geeignete Messprinzip verwendet werden, beispielsweise ein kapazitives, induktives oder resistives Messprinzip. Bei einer Ausgestaltung weist die Ermittlungseinrichtung wenigstens einen Sensor auf, der am oder im Körper des Patienten oder in der Nähe des Körpers angeordnet ist und ein die axiale Dislokation repräsentierendes Signal erzeugt. Bei einer weiteren Ausgestaltung wird der Stimulatorschaft unter Zuhilfenahme einer Einführhilfe in den Körper des Patienten eingeführt und nahe dem zu behandelnden Nerv positioniert. Als Einführhilfe kann eine Kanüle und/oder eine Kapillare verwendet werden, wobei prinzipiell zwischen unterschiedlichen Anlagetechniken unterschieden werden kann ("Kanüle über die Nadel", "Kanüle durch die Nadel", "Kanüle durch die Kapillare"). Der Stimulator kann als Katheter oder Modulationskatheter bezeichnet werden. Der Vorgang des Verschiebens bzw. des Anpassens oder Ausgleichens des Elektrodenaktivierungsmusters kann auch als Regelung oder Tracking bezeichnet werden. Das Verschieben des Elektrodenaktivierungsmusters kann auch als virtuelles Verschieben bezeichnet werden.

In Ausgestaltung der Erfindung ist die Ermittlungseinrichtung mit den mehreren Elektroden verbunden und eingerichtet zum Ermitteln von Impedanzen zwischen den Elektroden und damit eines Impedanzmusters des umgebenden Körpergewebes entlang der Längsachse des Stimulatorschafts, zum Ermitteln einer zeitlichen Änderung des Impedanzmusters, und zum Ermitteln der axialen Dislokation in Abhängigkeit der ermittelten zeitlichen Veränderung des Impedanzmusters. Bevorzugt ist das die Ermittlungseinrichtung zum Ermitteln von Impedanzen zwischen jeweils benachbarten Elektroden eingerichtet. Alternativ oder zusätzlich ist die Ermittlungseinrichtung zum Ermitteln von Impedanzen zwischen wenigstens einer der Elektroden und einer Hautelektrode eingerichtet. Der Stimulator, genauer: die mehreren Elektroden, fungieren bei dieser Ausgestaltung gleichsam als Sensor zum Erzeugen eines die axiale Dislokation repräsentierenden Signals, sodass zum Ermitteln der Dislokation kein separater Sensor notwendig ist. Dies stellt eine besonders vorteilhafte Ausgestaltung der Ermittlungseinrichtung dar. Dieser Ausgestaltung liegt die Erkenntnis zugrunde, dass die Impedanz von spezifischen Eigenschaften des umgebenden Körpergewebes abhängig ist und sich entlang der Längsachse des Stimulatorschafts in Abhängigkeit des umgebenden Körpergewebes verändert, d.h. einen Impedanzverlauf oder auch ein Impedanzmuster entlang der Längsachse ausbildet. Mit Impedanz ist der komplexe elektrische Widerstand des umgebenden Körpergewebes gemeint. Die Ermittlungseinrichtung approximiert den Impedanzverlauf entlang des Stimulatorschafts durch das ermittelte Impedanzmuster.

Bei einer axialen Dislokation des Stimulatorschafts bleibt der Impedanzverlauf erhalten, da er eine Eigenschaft des den Stimulatorschaft umgebenden Körpergewebes ist. Der Impedanzverlauf verschiebt sich jedoch in Bezug auf den Stimulatorschaft, genauer: die an dem Stimulatorschaft angeordneten Elektroden, entlang dessen Längsachse entgegen der Richtung der Dislokation. Zum Ermitteln der axialen Dislokation werden vorzugweise zwei zeitlich nacheinander ermittelte Impedanzmuster miteinander verglichen und abhängig von einem Vergleichsergebnis die axiale Dislokation ermittelt. Unter Ermitteln wird vorzugsweise ein Messen oder Erfassen verstanden. Bei einer Ausgestaltung werden die Impedanzen kontinuierlich oder in regelmäßigen oder unregelmäßigen zeitlichen Abständen ermittelt. Je schneller eine zeitliche Veränderung des Impedanzmusters ermittelt wird, desto schneller kann das System bzw. die Steuereinrichtung darauf reagieren und das Elektrodenaktivierungsmuster an die axiale Dislokation anpassen. Bei einer weiteren Ausgestaltung erfolgt das Verschieben des Elektrodenaktivierungsmusters, sobald eine zeitliche Änderung des Impedanzmusters ermittelt wird, die eine vorbestimmte Grenze überschreitet. Bei einer weiteren Ausgestaltung entspricht eine Auflösung des Impedanzverlaufs entlang der Längsachse einem Abstand benachbarter Elektroden. In einer alternativen Ausgestaltung werden Impedanzen zwischen nicht benachbarten Elektroden ermittelt, z.B. zwischen einer ersten Elektrode und einer übernächsten Elektrode, wodurch eine höhere Auflösung des Impedanzmusters möglich sein kann, und/oder es werden Impedanzen zwischen den Elektroden und einer weiteren separaten, insbesondere großflächigen, Basiselektrode ermittelt, die als Hautelektrode zum Aufbringen auf die Haut des Patienten gestaltet sein kann. Bei einer weiteren Ausgestaltung ist die Frequenz, mit der die Ermittlungseinrichtung ein Impedanzmuster ermittelt, veränderbar und insbesondere an äußere Umstände anpassbar. Zum Beispiel kann diese Frequenz, d.h. der zeitliche Abstand zwei aufeinanderfolgend ermittelter Impedanzmuster, verringert werden, wenn der Patient sich wahrscheinlich bewegen wird und dadurch die Gefahr einer Dislokation steigt.

In weiterer Ausgestaltung der Erfindung ist die Ermittlungseinrichtung eingerichtet zum Ermitteln der zeitlichen Änderung eines Impedanzspektrums der Impedanzen.

In weiterer Ausgestaltung der Erfindung ist die Ermittlungseinrichtung zum Ermitteln der zeitlichen Änderung des Impedanzmusters unter Berücksichtigung eines Realteils und/oder eines Imaginärteils der ermittelten Impedanzen eingerichtet. In weiterer Ausgestaltung der Erfindung ist die Ermittlungseinrichtung zum Ermitteln der zeitlichen Änderung des Impedanzmusters unter Berücksichtigung eines Betrags und/oder einer Phase der ermittelten Impedanzen eingerichtet. In weiterer Ausgestaltung der Erfindung ist die Ermittlungseinrichtung zum Ermitteln der zeitlichen Änderung des Impedanzmusters unter Berücksichtigung eines Spektrums der ermittelten Impedanzen eingerichtet. Hierdurch ist jeweils eine genaue Darstellung des Impedanzmusters bzw. einer Approximation des Impedanzverlaufs und dadurch ein genaues und zuverlässiges Ermitteln der axialen Dislokation möglich. Das Spektrum der ermittelten Impedanzen kann ein Frequenz- und/oder Amplitudenspektrum sein. Eine solche Analyse des Impedanzmusters kann als Impedanzspektroskopie bezeichnet werden. Bei einer Ausgestaltung werden mehrere der Eigenschaften Realteil, Imaginärteil, Betrag, Phase und Spektrum der Impedanzen zum Ermitteln der zeitlichen Änderung des Impedanzmusters gemeinsam berücksichtigt. Vorzugsweise ist die Ermittlungseinrichtung zum Ermitteln einer/der zeitlichen Änderung des Impedanzspektrums eingerichtet.

In weiterer Ausgestaltung der Erfindung sind die mehreren Elektroden zur Abgabe eines Messstroms für das Ermitteln der Impedanzen eingerichtet. Zudem ist die Steuereinrichtung zum Steuern der Abgabe des Messstroms eingerichtet. Hierdurch sind keine weiteren Elektroden und keine weitere separate Vorrichtung zum Erzeugen des Messstroms notwendig. Zudem können etwaige Wechselwirkungen des Messstroms mit dem umgebenden Körpergewebe auf einen lokalen Bereich um das distale Stimulatorende begrenzt werden, wodurch das umgebende Körpergewebe geschont wird. Zudem können aufgrund der vergleichsweise kleinen Abstände zwischen den Elektroden geringe Messströme verwendet werden. Bei einer Ausgestaltung erfolgt die Abgabe des Messstroms kontinuierlich in Form eines Gleichstroms oder Wechselstroms. Bei einer weiteren Ausgestaltung erfolgt die Abgabe des Messstroms diskret in regelmäßigen oder unregelmäßigen Abständen als Impulse.

In weiterer Ausgestaltung der Erfindung weist der Messstrom eine Stromstärke von maximal 10 mA auf, bevorzugt maximal 0,5 mA, besonders bevorzugt maximal 0,01 mA. Durch die geringe Stromstärke des Messstroms können Effekte auf das umgebende Körpergewebe und Wechselwirkungen mit dem umgebenden Körpergewebe vermieden oder zumindest gering gehalten werden.

In weiterer Ausgestaltung der Erfindung ist der Messstrom ein Wechselstrom, der eine Frequenz von wenigstens 10 Hz aufweist, bevorzugt wenigstens 1 kHz. Die Verwendung von Wechselstrom ermöglicht das Ermitteln einer echten Impedanz, d.h. einer Impedanz, deren Imaginärteil nicht null ist. Hierdurch lässt sich eine zeitliche Veränderung der Impedanz genauer und zuverlässiger ermitteln als bei Verwendung von Gleichstrom. Die Verwendung einer hohen Frequenz des Wechselstroms kann ein Risiko einer Stimulation motorischer Nerven verringern.

In weiterer Ausgestaltung der Erfindung ist der Messstrom ein Wechselstrom, der ein Frequenzspektrum, insbesondere ein Sweep, aufweist. Die Verwendung einer Mehrzahl von Frequenzen ermöglicht ein genaueres und/oder robusteres Messergebnis. Mit Frequenzspektrum ist gemeint, dass der Wechselstrom mehrere Frequenzen aufweist, der Messstrom also ein Multifrequenzsignal darstellt. Beispielsweise kann der Messstrom mehrere Frequenzkomponenten mit 10 Hz, 1kHz, 10 kHz und 1 MHz aufweisen. Dabei können die einzelnen Frequenzkomponenten gleichzeitig und/oder nacheinander auftreten. Unter einem Sweep versteht man in der Signalverarbeitung einen Wechselstrom, dessen Frequenz stetig einen vorgegebenen Bereich durchläuft. Der Bereich ist vorzugsweise durch eine minimale und eine maximale Frequenz definiert.

In weiterer Ausgestaltung der Erfindung ist die Ermittlungseinrichtung zum Ermitteln von Impedanzen, insbesondere von Impedanzspektren, auf Grundlage der abgegebenen elektrischen Stimuli eingerichtet. In einer Ausgestaltung ist die Ermittlungseinrichtung zum Ermitteln von Impedanzen über eine erste Gruppe von Elektroden bei Abgabe eines elektrischen Stimulus durch eine zweite Gruppe von Elektroden eingerichtet, wobei insbesondere die Elektroden der ersten Gruppe nicht in der zweiten Gruppe enthalten sind. Beispielsweise geben zwei Elektroden jeweils oder durch Wechselwirkung miteinander einen elektrischen Stimulus ab, der als Messsignal zum Ermitteln der Impedanzen dient. Speziell kann dieses Messsignal dann durch die übrigen, nicht zur Abgabe des Stimulus eingesetzten Elektroden zur Ermittlung von Impedanzen verwendet werden. In einer weiteren Ausgestaltung ist die Ermittlungseinrichtung zur Ermittlung von Impedanzen, insbesondere Impedanzspektren, sowohl auf Grundlage eines abgegebenen elektrischen Stimulus als auch auf Grundlage eines separaten Messstroms eingerichtet.

Die Erfindung betrifft zudem ein Verfahren zum Betreiben eines Systems gemäß der vorhergehenden Beschreibung. Das erfindungsgemäße Verfahren ist zum Betreiben eines Systems zur elektrischen Neurostimulation vorgesehen, wobei das System einen Stimulator mit einem längserstreckten Stimulatorschaft aufweist, der ein distales Stimulatorende mit mehreren Elektroden aufweist. Das Verfahren weist die folgenden Schritte auf: Ansteuern der mehreren Elektroden unter Ausbildung eines Elektrodenaktivierungsmusters mit aktivierten Elektroden und nicht aktivierten, d.h. deaktivierten, Elektroden; Ermitteln einer axialen Dislokation des Stimulatorschafts; Ansteuern der mehreren Elektroden, wobei das Elektrodenaktivierungsmuster in Abhängigkeit der ermittelten axialen Dislokation mittels einer veränderten Aktivierung und Deaktivierung der Elektroden axial verschoben wird, um die axiale Dislokation des Stimulatorschafts auszugleichen. Das erfindungsgemäße Verfahren ermöglicht die gleichen Vorteile wie das erfindungsgemäße System, sodass auf die entsprechenden Ausführungen verwiesen werden kann. Das zu dem erfindungsgemäßen System und dessen Ausgestaltungen Offenbarte gilt, mutatis mutandis, auch für das erfindungsgemäße Verfahren und Ausgestaltungen desselben.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Seitenansicht eine Ausführungsform eines erfindungsgemäßen Systems zur elektrischen Neurostimulation mit einem Stimulator, der einen Stimulatorschaft mit mehreren Elektroden aufweist, einer Steuereinrichtung und einer Ermittlungseinrichtung,
- Fig. 2: beispielhaft einen Impedanzverlauf eines den Stimulatorschaft umgebenden Körpergewebes entlang einer Längsachse des Stimulatorschafts,
- Fig. 3, 4: in schematischer Seitenansicht den Stimulator mit einem Elektrodenaktivierungsmuster und einem ermittelten Impedanzmuster vor Auftreten einer axialen Dislokation (Fig. 3) und den Stimulator mit einem verschobenen Elektrodenaktivierungsmuster nach Auftreten der axialen Dislokation (Fig. 4).

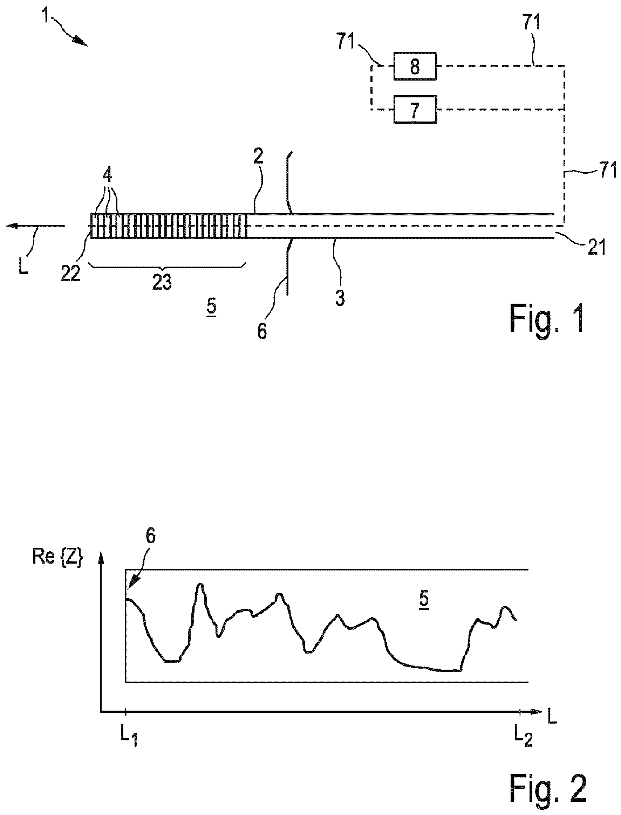

Gemäß Fig. 1 ist ein System 1 zur elektrischen Neurostimulation vorgesehen. Das System 1 weist einen Stimulator 2 auf.

Bei der gezeigten Ausführungsform ist der Stimulator 2 als Katheter gestaltet und dient im Speziellen als Schmerzkatheter bei einer Schmerztherapie, die auch als peripherer Nervenblock bezeichnet wird.

Der Stimulator 2 weist einen Stimulatorschaft 3 und mehrere Elektroden 4 auf. Der Stimulatorschaft 3 ist zwischen einem proximalen Stimulatorende 21 und einem distalen Stimulatorende 22 entlang einer Längsachse L längserstreckt.

Die Elektroden 4 sind entlang der Längsachse L des Stimulatorschafts 3 zueinander benachbart und im Bereich des distalen Stimulatorendes 22 angeordnet. Der besagte Bereich wird im Folgenden auch als distaler Endabschnitt 23 bezeichnet.

In der Verwendung des Systems 1 wird der Stimulator 2 mit dem distalen Endabschnitt 23 voran im Körpergewebe 5 eines Patienten in unmittelbarer Nähe zu einem zu betäubenden Nerv positioniert. Zur Anlage des Stimulators 2 wird der Stimulatorschaft 3 gegebenenfalls unter Zuhilfenahme einer Einführhilfe im Körpergewebe 5 positioniert. Mittels der Elektroden 4 können elektrische Stimuli an den zu betäubenden Nerv abgegeben werden. Die Stimuli dienen der Schmerzreduktion.

Das System 1 weist zudem eine Steuereinrichtung 7 auf, die mit den Elektroden 4 über eine elektrische Signalleitung 71 verbunden ist. Die Steuereinrichtung 7 ist dazu eingerichtet, über die Signalleitung 71 die Elektroden 4 unabhängig voneinander zu aktivieren und zu deaktivieren. Die Signalleitung 71 verläuft vorliegend entlang und im Inneren des Stimulatorschafts 3. Die Elektroden 4 nehmen jeweils einen von zwei Zuständen (aktiviert/deaktiviert) ein und bilden dadurch entlang der Längsachse L ein Elektrodenaktivierungsmuster 41 aus. Der über die Elektroden 4 an das Körpergewebe 5 bzw. den Nerv abgegebene elektrische Stimulus ist charakteristisch für das Elektrodenaktivierungsmuster 41. Durch eine entsprechende Ansteuerung der Elektroden 4 steuert die Steuereinrichtung 7 somit den an das Körpergewebe 5 bzw. den Nerv abgegebenen elektrischen Stimulus.

Das System 1 weist weiter eine Ermittlungseinrichtung 8 auf. Diese ist vorliegend über die elektrische Signalleitung 71 mit der Steuereinrichtung 7 verbunden ist. Zudem ist die Ermittlungseinrichtung 8 über die Signalleitung 71 mit den Elektroden 4 verbunden.

Die Ermittlungseinrichtung 8 ist dazu eingerichtet, Impedanzen Z zwischen jeweils benachbart angeordneten Elektroden 4 zu ermitteln, wobei die Impedanzen Z - im angelegten Zustand des Stimulators 2 - maßgeblich durch das den distalen Endabschnitt 23 umgebende Körpergewebe 5 bestimmt werden. Zur Ermittlung der Impedanzen Z steuert die Steuereinrichtung 7 die Elektroden 4 zur Abgabe eines Messstroms an. Der Messstrom kann ein breitbandiges Signal sein, insbesondere ein Signal bestehend aus mehreren Frequenzen oder ein Sweep, beispielsweise mit einer maximalen Stromstärke von z.B. 10 mA und einem Frequenzbereich zwischen 10 Hz und 1 MHz. Um ungewollte Nervenreaktionen zu vermeiden, kann der Frequenzbereich auf hochfrequente Signale oberhalb von z.B. 1 kHz eingeschränkt werden. Alternativ kann ein breitbandiges Puls-Signal als Messstrom verwendet werden. In einer Ausgestaltung kann ein über die Elektroden 4 abgegebener elektrischer Stimulus als Messsignal zur Ermittlung von Impedanzen Z dienen, sofern der elektrische Stimulus hierfür geeignet ist, beispielsweise als Puls-Signal ausgestaltet ist.

Die Impedanz Z verändert sich über das Körpergewebe 5 hinweg abhängig von den lokalen Eigenschaften des Körpergewebes 5. Fig. 2 zeigt einen beispielhaften Impedanzverlaufs Z entlang der Längsachse L im Körper eines Patienten. Der Punkt L1 auf der die Längsachse L repräsentierenden Abszisse entspricht vorliegend einer Eintrittsstelle des Stimulatorschafts 3 in das Körpergewebe 5. Vom Punkt L1 aus entlang der Längsachse L bis zu einem Punkt L2 verändert sich die Impedanz Z abhängig vom Ort entlang der Längsachse L kontinuierlich, in Fig. 2 repräsentiert durch den Realteil Re{Z} der Impedanz. Ersichtlich unterscheiden sich unterschiedliche Abschnitte entlang der Längsachse L hinsichtlich der dort ermittelten Impedanz Z.

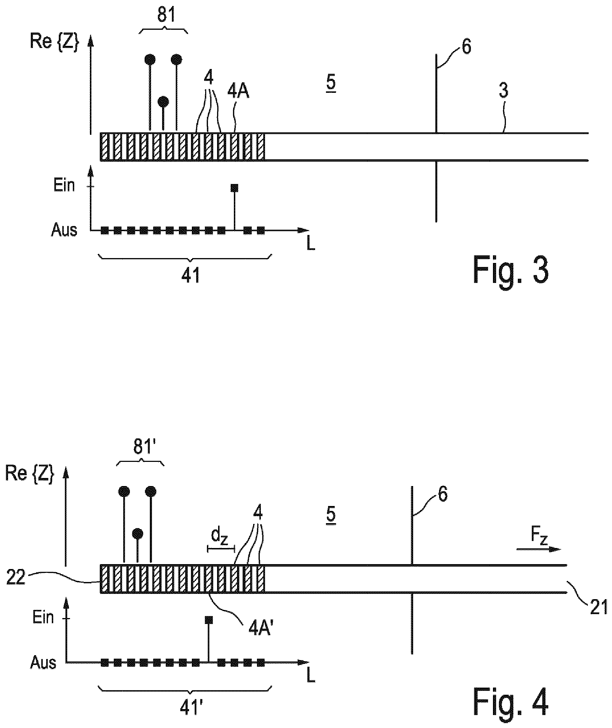

Diese Eigenschaft des Körpergewebes 5 macht sich das System 1 zur Ermittlung einer axialen Dislokation zunutze. Im Folgenden wird unter Verweis auf die Fig. 3 und 4 ein Verfahren zum Anpassen der Abgabe elektrischer Stimuli an eine axiale Dislokation beschrieben.

Der Stimulatorschaft 3 ist mit dem distalen Endabschnitt 23 in der Nähe des vorliegend nicht näher gezeigten Nervs positioniert. Der Stimulatorschaft 3 ist zumindest im Bereich des distalen Endabschnitts 23 von Körpergewebe 5 umgeben. Zur Erzeugung eines elektrischen Stimulus oder mehrerer aufeinanderfolgender elektrischer Stimuli oder eines kontinuierlichen elektrischen Stimulus steuert die Steuereinrichtung 7 die Elektroden 4 an. Abhängig davon, welche Elektroden 4 die Steuereinrichtung 7 aktiviert und welche sie deaktiviert, ergibt sich ein Elektrodenaktivierungsmuster 41. Im gezeigten Beispiel ist lediglich die - in Längsrichtung L gesehen - drittletzte Elektrode 4A aktiviert. Zudem steuert die Steuereinrichtung 7 alle Elektroden 4 zur Abgabe eines Messstroms an. Der Messstrom ist geringer als der zur Erzeugung des elektrischen Stimulus abgegebene Strom einer jeweiligen Elektrode 4. Die Ermittlungseinrichtung 8 ermittelt ein Impedanzmuster 81, dessen Realteil Re{Z} in Fig. 3 dargestellt ist. Das Impedanzmuster 81 setzt sich aus den einzelnen, zwischen den benachbarten Elektroden 4 ermittelten Impedanzen zusammen. Wie gezeigt, kann die Impedanz jeden beliebigen Wert zwischen einem maximalen und einem minimalen Impedanzwert annehmen, abhängig von einer Auflösung der Ermittlungseinrichtung 8. Das Impedanzmuster 81 entlang der Längsachse L ist charakteristisch für die Position des Stimulatorschafts 3 relativ zum Körpergewebe 5 und damit relativ zu dem zu betäubenden Nerv.

Wirkt nun eine Kraft Fz auf den Stimulatorschaft 3, verändert dieser seine axiale Position in Bezug auf das Körpergewebe 5 (axiale Dislokation). In dem vorliegenden Beispiel wird der Stimulatorschaft 3 aus dem Körper des Patienten herausgezogen. Infolgedessen verändern sich auch die Positionen der einzelnen Elektroden 4 in Bezug auf das Körpergewebe 5, sodass das durch die Steuereinrichtung 7 erzeugte Elektrodenaktivierungsmuster 41 nicht mehr den gewünschten schmerzreduzierenden Effekt erzielen kann. Anders ausgedrückt bilden die Elektroden 4 in Bezug auf das den Stimulatorschaft 3 umgebende Körpergewebe 5 nicht mehr das gleiche Elektrodenaktivierungsmuster 41 aus.

Nach erfolgter Dislokation des Stimulatorschafts 3 ermittelt die Ermittlungseinrichtung 8 erneut ein Impedanzmuster, ein sog. verschobenes Impedanzmuster 81'. Das verschobene Impedanzmuster 81' ist in Bezug auf die Längsachse L relativ zum ursprünglich ermittelten Impedanzmuster 81 verschoben. Im gezeigten Beispiel ist das verschobene Impedanzmuster 81' gegenüber dem ursprünglichen Impedanzmuster um den Abstand dz in Richtung des distalen Stimulatorendes 22 verschoben. Abgesehen von der Verschiebung entlang der Längsachse L sind das ursprüngliche Impedanzmuster 81 und das verschobene Impedanzmuster 81' aber identisch.

Durch Vergleichen des ursprünglichen Impedanzmusters 81 mit dem verschobenen Impedanzmuster 81' kann die Ermittlungseinrichtung den Abstand dz ermitteln. Der Abstand dz repräsentiert die axiale Dislokation. Insofern ist der Abstand dz gleich der axialen Dislokation oder weist zumindest eine nur geringe Abweichung von der tatsächlichen Dislokation auf.

Damit das Elektrodenaktivierungsmuster 41 weiterhin den gewünschten Effekt im Körpergewebe 5 erzielen kann, wird es ebenfalls um den Abstand dz verschoben, konkret in Richtung des distalen Stimulatorendes 22. Zu diesem Zweck übermittelt die Ermittlungseinrichtung 8 den Abstand dz an die Steuereinrichtung 7 über die Signalleitung 71. Die Steuereinrichtung 7 verändert die Ansteuerung der Elektroden 4 abhängig von der Dislokation, d.h. abhängig vom Abstand dz. Im gezeigten Beispiel deaktiviert die Steuereinrichtung 7 die zuvor aktivierte drittletzte Elektrode 4A und aktiviert stattdessen die fünftletzte Elektrode 4A'. Die in dieser Weise durch die Steuereinrichtung veränderte, d.h. neu eingestellte, Aktivierung und Deaktivierung der Elektroden 4 bildet ein verschobenes Elektrodenaktivierungsmuster 41' aus. Abgesehen von der Verschiebung entlang der Längsachse L sind das ursprüngliche Elektrodenaktivierungsmuster 41 und das verschobene Elektrodenaktivierungsmuster 41' identisch. Mit anderen Worten bildet das Elektrodenaktivierungsmuster 41 die Bewegung des Impedanzmusters 81 entlang der Längsachse L nach. Das Elektrodenaktivierungsmuster wird dem Impedanzmuster nachgeführt.

Falls der Abstand dz ein ganzzahliges Vielfaches des Abstands zwischen zwei benachbarten Elektroden 4 beträgt, weist das verschobene Elektrodenaktivierungsmuster 41' die gleiche Position in Bezug auf das umgebende Körpergewebe 5 auf wie das ursprüngliche Elektrodenaktivierungsmuster 41.

Im andern Fall steuert die Steuereinrichtung 7 die Elektroden 4 zur Ausbildung eines verschobenen Elektrodenmusters 41' derart an, dass die Position des verschobenen Elektrodenaktivierungsmusters 41' in Bezug auf das Körpergewebe 5 den kleinsten Abstand zur Position des ursprünglichen Elektrodenaktivierungsmusters 41 aufweist.

Die Steuereinrichtung 7 arbeitet also daraufhin, dass das Elektrodenaktivierungsmuster 41 unabhängig von bzw. trotz einer Dislokation des Stimulatorschafts 3 seine Position relativ zum Körpergewebe 5 beibehält oder zumindest im Wesentlichen beibehält. Dadurch ist auch der mittels der Elektroden 4 abgegebene elektrische Stimulus gleich oder zumindest ähnlich und bewirkt den gleichen oder zumindest einen ähnlichen Effekt im Körpergewebe 5.

## Patentansprüche

1. System (1) zur elektrischen Neurostimulation, aufweisend
einen Stimulator (2) mit einem längserstreckten Stimulatorschaft (3), der ein distales Stimulatorende (22) mit mehreren Elektroden (4) aufweist, die entlang einer Längsachse (L) des Stimulatorschafts (3) benachbart angeordnet und zur Abgabe elektrischer Stimuli an ein den Stimulatorschaft (3) umgebendes Körpergewebe (5) eingerichtet sind,
eine Steuereinrichtung (7), die mit den mehreren Elektroden (4) verbunden und zum Steuern der Abgabe der elektrischen Stimuli eingerichtet ist, wobei die mehreren Elektroden (4) mittels der Steuereinrichtung (7) voneinander unabhängig aktivierbar und deaktivierbar und damit zur Ausbildung entlang der Längsachse (L) unterschiedlicher Elektrodenaktivierungsmuster (41, 41') ansteuerbar sind,
**dadurch gekennzeichnet, dass** eine Ermittlungseinrichtung (8) vorhanden und zum Ermitteln einer axialen Dislokation des Stimulatorschafts (3) eingerichtet ist,
wobei die Steuereinrichtung (7) mit der Ermittlungseinrichtung (8) verbunden und zum axialen Verschieben des Elektrodenaktivierungsmusters (41, 41') mittels einer Veränderung der Aktivierung und Deaktivierung der Elektroden (4) in Abhängigkeit der ermittelten axialen Dislokation eingerichtet ist, um die Abgabe der elektrischen Stimuli an die axiale Dislokation des Stimulatorschafts (3) örtlich anzupassen.

2. System (1) nach Anspruch 1, **dadurch gekennzeichnet, dass** die Ermittlungseinrichtung (8) mit den mehreren Elektroden (4) verbunden und eingerichtet ist
zum Ermitteln von Impedanzen (Z) zwischen Elektroden (4) und damit eines Impedanzmusters (81, 81') des umgebenden Körpergewebes (5) entlang der Längsachse (L) des Stimulatorschafts (3),
zum Ermitteln einer zeitlichen Änderung des Impedanzmusters (81, 81'), und
zum Ermitteln der axialen Dislokation in Abhängigkeit der ermittelten zeitlichen Veränderung des Impedanzmusters (81, 81'),
insbesondere zum Ermitteln von Impedanzen (Z) zwischen jeweils benachbart angeordneten Elektroden (4).

3. System (1) nach Anspruch 2, **dadurch gekennzeichnet, dass** die Ermittlungseinrichtung (8) eingerichtet ist zum Ermitteln der zeitlichen Änderung eines Impedanzspektrums der Impedanzen (Z).

4. System (1) nach Anspruch 2 oder 3, **dadurch gekennzeichnet, dass** die Ermittlungseinrichtung (8) eingerichtet ist zum Ermitteln der zeitlichen Änderung des Impedanzmusters (81, 81') unter Berücksichtigung eines Realteils und/oder eines Imaginärteils der ermittelten Impedanzen (Z).

5. System (1) nach einem der Ansprüche 2 bis 4, **dadurch gekennzeichnet, dass** die Ermittlungseinrichtung (8) eingerichtet ist zum Ermitteln der zeitlichen Änderung des Impedanzmusters (81, 81') unter Berücksichtigung eines Betrags und/oder einer Phase der ermittelten Impedanzen (Z).

6. System (1) nach einem der Ansprüche 2 bis 5, **dadurch gekennzeichnet, dass** die Ermittlungseinrichtung (8) eingerichtet ist zum Ermitteln der zeitlichen Änderung des Impedanzmusters (81, 81') unter Berücksichtigung eines Spektrums der ermittelten Impedanzen (Z).

7. System (1) nach einem der Ansprüche 2 bis 6, **dadurch gekennzeichnet, dass** die mehreren Elektroden (4) zur Abgabe eines Messstroms für das Ermitteln der Impedanzen (Z) eingerichtet sind, und dass die Steuereinrichtung (7) zum Steuern der Abgabe des Messstroms eingerichtet ist.

8. System (1) nach Anspruch 7, **dadurch gekennzeichnet, dass** der Messstrom eine Stromstärke von maximal 10 mA, bevorzugt maximal 0,5 mA, besonders bevorzugt maximal 0,01 mA, aufweist.

9. System (1) nach Anspruch 7 oder 8, **dadurch gekennzeichnet, dass** der Messstrom ein Wechselstrom ist, der eine Frequenz von wenigstens 10 Hz, bevorzugt wenigstens 1 kHz, aufweist.

10. System (1) nach einem der Ansprüche 7 bis 9, **dadurch gekennzeichnet, dass** der Messstrom ein Wechselstrom ist, der ein Frequenzspektrum, insbesondere ein Sweep, aufweist.

11. System (1) nach einem der vorhergehenden Ansprüche, **dadurch gekennzeichnet, dass** die Ermittlungseinrichtung (8) zum Ermitteln von Impedanzen (Z) auf Grundlage der abgegebenen elektrischen Stimuli eingerichtet ist, insbesondere zum Ermitteln von Impedanzen (Z) über eine erste Gruppe von Elektroden (4) bei Abgabe eines elektrischen Stimulus durch eine zweite Gruppe von Elektroden (4), wobei insbesondere die Elektroden (4) der ersten Gruppe nicht in der zweiten Gruppe enthalten sind.

12. Verfahren zum Betreiben eines Systems (1) zur elektrischen Neurostimulation, wobei das System (1) einen Stimulator (2) mit einem längserstreckten Stimulatorschaft (3) aufweist, der ein distales Stimulatorende (22) mit mehreren Elektroden (4) aufweist, wobei das Verfahren die Schritte aufweist:
Ansteuern der mehreren Elektroden (4) unter Ausbildung eines Elektrodenaktivierungsmusters (41, 41') mit aktivierten und nicht aktivierten Elektroden (4);
Ermitteln einer axialen Dislokation des Stimulatorschafts (3);
Ansteuern der mehreren Elektroden (4), wobei das Elektrodenaktivierungsmuster (41, 41') in Abhängigkeit der ermittelten axialen Dislokation mittels einer veränderten Aktivierung und Deaktivierung der Elektroden (4) axial verschoben wird, um die axiale Dislokation des Stimulatorschafts (3) auszugleichen.
